# EUROPEAN PATENT APPLICATION

(11) **EP 1 625 877 A2**
(43) Date of publication of application: **15.02.2006**
(21) Application number: 04077265.9
(22) Date of filing: 09.08.2004
(51) Int. Cl.: A61Q 11/00, A61K 8/64, A61K 8/98, A23C 9/142, A23L 2/52

(54) **Functional milk fraction**

(71) Applicant: Friesland Brands B.V., 7943 PE Meppel (NL)
(72) Inventor: Bos, Rolf, 8032 LC Zwolle (NL); Beernink, Roy, 7451 VB Holten (NL); Oudeman, Evert, 9404 EN Assen (NL)
(74) Representative: Winckels, Johannes Hubertus F.

(57) **Abstract**

The present invention relates to a functional milk fraction which can be used to reduce negative effects associated with the oral uptake of acidic foods and beverages, to a method of isolating said specific milk fraction and to the use of this milk fraction in compositions for oral care. The functional milk fraction can be used in edible products, especially to counteract the negative effects of the consumption of acidic foods and beverages.

## Description

The present invention relates to a functional milk fraction which can be used to reduce negative effects associated with the oral uptake of acidic foods and beverages, to a method of isolating said specific milk fraction and to the use of this milk fraction in compositions for oral care. The functional milk fraction can be used in edible products, especially to counteract the negative effects of the consumption of acidic foods and beverages or of otherwise caused acidic conditions in the mouth.

It is well known that any beverage and/or foodstuff with a pH of less than 5.5 can damage the teeth by eroding the tooth enamel. For instance, Millward *et al*. (1994, Int. J. Paed. Dent. 4, 151-157) associated the presence of dental erosion in children with the consumption of acidic beverages and fruit juices. Over the last decades, the prevalence of dental erosion seems to have increased (Nunn, 1996, Eur. J. Oral Sci. 104, 156-161), presumably because of the increased popularity of acidic beverages, like fruit juices and carbonated soft drinks; these drinks are becoming increasingly popular among all ages of consumers and are replacing tea and coffee as the most popular beverage products.

Several steps have been made to modify the composition of acidic foodstuffs with the aim of reducing the observed negative effects on oral health. The addition of compounds or mixtures supplying minerals like calcium and/or phosphate salts has attracted much attention (see for example WO-A-01/52796, WO-A-01/72144, EP-A-0 551 398, US-A-3,968,263, GB-A-1,516,525).

WO-A-97/30601 and WO-A-99/08550 disclose acid-based liquid compositions having reduced tooth erosion properties containing a calcium compound and an acidulant in which compositions calcium is present in the range of 0.3 to 0.8 mole per ml of acidulant and the pH of the composition is from 3.5 to 4.5.

WO 00/13531 discloses the use of viscosity modifying polymer materials, commonly used as thickening agents, stabilisers and emulsifiers, for oral use to alleviate or inhibit tooth damage associated with the consumption of acidic compositions. In EP-A-0 874 558, the use of viscosity-enhancing polymers to prevent tooth erosion is claimed.

A problem with supplying calcium and phosphate salts to acidic beverages is that these compounds cause changes that have an effect on the flavour as well as on the pH of these drinks depending on the salts and concentration used (*cfr*. Grenby, 1996, Eur. J. Oral Sci. 104, 207-214).

Another obvious substance to be added as a food ingredient would be fluoride. Analogous to its anti-cariogenic properties, several researchers (see e.g. Gedalia *et al*., 1981, Caries Res. 15, 103-108 and Sorvari *et al*. 1994, 28, 227-232) have demonstrated an anti-erosive effect of fluoride when present in acidic drinks. The concentration of fluoride used in those studies ranges from 2 to as high as 50 ppm, which may raise concerns about the risk of overconsumption through this route and would therefore not be permitted for use as a food ingredient. For this reason, it is an object of the present inventors to identify factors in natural food products that can protect teeth against caries and other challenges.

There are indications that milk or cheese may contain factors that are beneficial for oral health. For example, a comparative study in the UK showed that children who drank milk had lower caries prevalence than children who did not drink any milk (O'Sullivan *et al.* 2000, ASDC J. Dent. Child. 67(3), 186-192).

Also processed cheese has been shown to prevent demineralisation and enhance remineralisation of enamel and root lesions in a human *in situ* caries model (Jensen *et al*. 1990, Am. J. Dent. 3(5), 217-223). Some of the effects of milk can be ascribed to the calcium and phosphorous levels present in milk. Besides minerals, other milk components, such as casein or phosphopeptides derived thereof. (Reynolds, 1997, J. Dent. Res. 76, 1587-1595) and proteose-peptone fractions (Grenby *et al*., 2001, J. Dentistry 29(2), 83-92) have been reported to reduce enamel solubility as well.

Further, the addition of whey minerals to fruit juice reduced the enamel softening effect of this beverage (Thomason 1990, Caries Res. 24, 334-336).

In summary, it is known that milk products and constituents thereof have or at least may have a positive effect on teeth. Particularly, positive effects have been associated with the minerals present in milk and especially the calcium and phosphate ions and/or salts, with the casein fraction, with phosphopeptides derived from casein and with the proteose-peptone fraction.

The present inventors have found that a specific milk fraction and especially a specific whey fraction has a protective effect on teeth. The activity of this milk fraction is not based on the presence of calcium and/or phosphate minerals, casein, phosphopeptides derived from casein or proteose/peptone.

More particularly, the inventors isolated a naturally present milk fraction and especially a low molecular weight milk fraction that was found to reduce the negative effects on oral health associated with the consumption of acidic compositions. The particular milk fraction can preferably be administered to compositions and especially to acidic compositions, such as foodstuffs, for oral use, in particular to beverages.

In a first aspect, the present invention relates to a method for isolating a dental or tooth-erosion reducing composition or a composition reducing the negative effects of acidic compounds on oral health, comprising contacting of milk with hydroxyapatite; and eluting any materials attached to the hydroxyapatite.

Preferably, the method comprises isolating the whey fraction of milk and contacting of this whey fraction with hydroxyapatite. In especially preferred embodiments milk or whey is subjected to a protein-removing step, preferably to ultrafiltration, and the protein-free fraction is contacted with hydroxyapatite. That is, the isolated milk fraction having the dental erosion reducing activity is not the casein fraction of milk nor the whey protein or the proteose-peptone containing composition.

In preferred embodiments, the milk or whey fraction is demineralised and/or subjected to a lactose removing step before contacting it with hydroxyapatite.

In a second aspect, the invention relates to a tooth erosion reducing composition obtainable by the method of the invention. This tooth erosion reducing composition is not a mineral fraction, a casein based composition, nor the whey proteins including the proteose-peptone fraction of milk; said activity is not due to the presence of calcium and/or phosphate in the whey fraction.

The active fraction of milk can be prepared by the following method. Milk, preferably after removal of the fat fraction, or more preferred the whey fraction of milk, is subjected to a protein removing step. This protein free product is concentrated.

In a preferred embodiment the milk or whey is subjected to ultrafiltration, and the tooth reducing composition is present in the permeate. From the permeate or more in general from the protein-free fraction, the anti-erosive fraction can be further isolated, preferably using demineralisation and/or lactose removal steps.

In a preferred embodiment, the protein-fee fraction if whey is subjected to a calcium-phosphate removing step before the concentrating step. Very suitably, a whey ultrafiltration permeate is treated with alkali to a slightly alkaline pH (pH ≥ 7.5, preferably between 8.0 and 9.0) and moderately heated to a temperature between 45 and 60 °C for about 30-90 minutes, such that a precipitate is formed. the precipitate is removed, e.g. by microfiltration. The anti-corrosive factor is present in the precipitate. After acidification to a pH below 4, the precipitate is subjected to size exclusion chromatography (preferably using gel filtration using Sephadex G-25) to remove salts. The demineralized fraction of the precipitate contains the anti-erosive components.

In a further aspect, the present invention relates to a food composition and preferably a beverage, and more preferably an acidic beverage comprising the tooth reducing composition of the invention.

In yet a further embodiment, the present invention relates to the use of a whey fraction, not being milk minerals or lactose, for the manufacture of a composition to reduce tooth erosion. Preferably, the composition manufactured is based on an acidic food product, and more preferably on a beverage such as an acidic beverage.

More in detail, the invention relates to the use of the low molecular weight nitrogen fraction of milk or whey for the manufacture of a composition to reduce tooth erosion. The low molecular weight nitrogen fraction is that fraction not being the lactose and mineral fraction having molecular weights smaller than 20 kDa and preferably smaller than 10 kDa.

As used herein, "negative effects on oral health" or "dental or tooth erosion" is defined as loss, softening and/or demineralisation of tooth substance. Particularly, such loss of tooth substance occurs by direct action of acid on the tooth substance. Such acid may, for example, be present in the oral cavity either naturally, e.g. through chronic regurgitation (occurring for instance in association with anorexia nervosa, bulimia nervosa and/or gastrointestinal disturbances) or by consumption of acidic foods and beverages.

The invention is advantageously applied in acidic compositions, in particular foodstuffs and especially beverages, containing natural and/or added acidulants, including fruit juices The acid composition may contain organic and/or inorganic acids and may be supplemented with vitamins such as ascorbic acid. Preferred acidulants include potable acids such as citric, maleic, lactic, phosphoric, acetic and tartaric acids and mixtures thereof. The acids may be present in their non-dissociated form or, alternatively, as their respective salts; for example potassium or sodium hydrogen phosphate or potassium or sodium dihydrogen phosphate salts.

Not only reduces the low molecular weight fraction of milk, and especially the low molecular weight nitrogen fraction of whey containing composition, the erosive potential of acidic beverages and food products, but also does this fraction in general not adversely affect the characteristics of the product to which it is added , like taste and pH.

The invention is applicable to all products for oral consumption or use by a mammal, preferably a human, including acidic foods and beverages. .Examples of acidic beverages to which the tooth erosion reducing composition of the invention can be added, are still and carbonated alcoholic and non-alcoholic beverages (e.g., fruit drinks and cola beverages).

The composition of the invention can also be used in products like tooth paste, mouth water, chewing gum or other known oral care compositions.

The food products including beverages, to which the active composition of the invention can be added, may be unsweetened, or sweetened with natural sugars or artificial sweeteners such as saccharine, aspartyl phenyl alanyl methyl ester, or other sweeteners known in the art. Compositions may also contain other conventional additives such as preservatives (sodium benzoate, sorbic acid, sodium metabisulfite, etc), vitamins, anti-oxidants, minerals, stabilizers, acidity regulators, thickeners, flavourings, colourings and carbon dioxide and functional health compounds.

Preferably, the food and beverage compositions comprise from about 0.01% to about 5%, more preferably from about 0.1% to about 2% of the whey fraction of the invention, based on weight of the total composition.

The effective pH of compositions for oral use according to the invention will vary according to type or product, acid content and desired organoleptic properties. Generally, acidic compositions to which the active milk fraction is added have a pH of less than about 5.5, preferably from about 2 to about 5 and most preferably from about 2.7 tot about 4.5.

As mentioned herein-above the tooth erosion reducing composition can also be used to neutralize tooth eroding effects caused by an acidic environment in the mouth caused by other means than the uptake of eroding acidic foods and beverages.

In such a case compositions containing the active factor can be used including dairy products, like yoghurt, fruit yoghurt, whey and butter milk.

As said herein-above, the present inventors have found a particular fraction of milk and especially a particular whey fraction that apparently under conditions in the mouth when a person or animal consumes an acidic food product, and especially an acidic beverage protects the teeth. These whey components have a high binding affinity to the major component of tooth enamel, *viz*. hydroxyapatite. This binding behaviour is used in the method of the invention, in that the fraction in milk that binds to hydroxyapatite is used in the reduction of dental erosion. The composition that binds to hydroxyapatite can be eluted using e.g. phosphate buffer, and especially 0.5M phosphate buffer. If desired, the eluted product can be dialysed to remove any salts, and/or freeze dried or otherwise concentrated.

As said, the anti-erosive component(s) of the present invention are present in the low-molecular fraction of milk and can be isolated therefrom. In a preferred embodiment, the anti-erosive compound can be isolated from the permeate of an commonly used ultrafiltration method separating the casein and/or whey protein fraction from the low molecular weight whey fraction. This means that the anti-erosive composition can also be isolated from either commercially available or otherwise prepared whey powder containing the low molecular weight components..

The present invention will now be described in further detail in the following non-limiting examples. In case percentages are mentioned, these percentages are drawn - as anywhere else in this description - on the weight of the solids in the final composition, unless otherwise indicated.

From the examples it will become clear that the anti-erosive action of whey can be surprising attributed to the permeate low molecular weight fraction of milk-both milk and whey permeate-and not to the calcium and phosphorous present in permeate. Additional processing like demineralisation and delactosing are not harmfull to the effect. It has been possible to concentrate the anti-erosive effect of the permeate by elution after binding to a hydroxyapatite column. In addition, precipitation can be used to concentrate the components from permeate responsible for the anti-erosive activity as well as well-known isolation processes like chromatography and filtration. The milk permeate and whey permeate are effective against tooth erosion due to acidity. The anti-erosive components can be easily concentrated in well-known processes like demineralisation, delactosing, chromatography, precipitation, and filtration.

In the working examples, the compositions were tested for reduction of tooth erosion using the following tests. Acidic compositions, for example fruit juice beverages and soft drinks (e.g., cola beverages), may cause the consumer of the beverage to experience dental erosion. Such dental erosion is caused because of the acidic nature of the beverage composition, i.e., compositions exhibiting a pH of about 5.5 or lower. As such, it is important to measure the erosive properties (or lack thereof) of acidic compositions. Hereinbelow, the erosive potential of several acidic composition with and without the isolated whey fraction is determined using the following method.

Freshly extracted bovine incisors with removed roots were stored in tap water at 4°C. First, the teeth were optically selected for the absence of carious lesions or other observable defects. Second, these were flattened and then cut in rectangular blocks of intact enamel (3 mm x 3 mm) by using a water-cooled diamond saw (Buehler, 11-4243). The resulting enamel block, with the exception of the enamel surface exposed to the oral cavity, was embedded in PMMA (Autoplast polymer, De Trey). Minimum enamel thickness was 1.0 mm.

Before use, enamel blocks were polished on emery-paper (with an average enamel loss of 100 µm). Enamel blocks were placed in 1 ml acid solution (50 mM citric acid, 1 mM NaN₃, 0.4 mM CaCl₂·2H₂O, 0.4 mM KH₂PO₄, pH 3.0) with and without the product according to the invention for different times, while shaking at room temperature. After incubation, enamel blocks were rinsed with 4.4 ml distilled water and 0.6 ml lanthanum-HCl (10 mM LaCl₃, 50 mM HCl). This solution was analysed by atomic absorption spectrophotometry (AAS) for its calcium concentration, the difference in calcium content being a representation of actual mineral loss. These proceedings were subsequently repeated twice for each enamel block in the equal test solution.

The first acid attack was intended to remove a lubricant surface layer on the enamel block and was not used for further calculations. Calcium losses of next two acid attacks were measured. After correction for dilution, values were averaged to determine calcium loss (ppm).

### Example 1

150 mg Domovictus-100 whey powder (produced by BorculoDomo Ingredients, the Netherlands; comprising about 74% lactose, 12% whey protein, 8% minerals, 3% NPN nitrogen compounds, 1% moisture; and 1% fat) was dissolved in 2 ml water. 2 ml of EDTA (0.2M) was added to remove peptide/protein bound calcium. The decalcified proteins and peptides were recovered from this solution by passing this solution on a Millipore C-18 SPE column. After washing with water, proteins and peptides were recovered by eluting the column with acetonitrile (60%). After drying in a rotavapor, residue was dissolved in 2 ml water.

Hydroxyapatite was diluted with water to a dry matter content of 6%.

Adsorption is performed by addition of 800 µl buffer (either 5 mM Tris/HCl + 41 mM NaCl (pH=7.0); 5 mM MES/NaOH + 41 mM NaCl (pH=6.0) 5 mM Acetate + 41 mM NaCl (pH=5.0) 5 mM Acetate + 41 mM NaCl (pH=4.0); 100 µl protein/peptide solution; and 100 µl hydroxyapatite solution. Adsorption was performed at 37°C in a shaking incubator. At the end of the incubation (16 hours, overnight), supernatant was removed. The hydroxyapatite was eluted using 0.5 M phosphate buffer. It was found that 2.5 mg hydroxyapatite was able to bind about 50 µg whey material.

### Example 2

To concentrate the active anti-erosive ingredients from permeate, a whey fraction with a high binding affinity for hydroxyapatite (a major component of tooth enamel) was isolated. This whey fraction was isolated from Domovictus-100, described in Example 1.

Particularly, 659.4 mg Domovictus-100, 4 ml hydroxyapatite suspension, 12.5 ml water and 0.1M acetate buffer pH=6 were mixed and incubated at 25°C in a shaking stove for 20 hours. Then the mixture was centrifuged and washed with water (one time). Phosphate buffer (25 ml, 0.5M, pH = 7.0) was subsequently added to the precipitate and during the next 4 hours desorption took place at room temperature. The supernatant was collected and dialysed with water during 24 hours. After this dialysis, the fraction was freeze dried.

The freeze-dried product was tested for anti-erosive activity and was found to have distinctive anti-erosive effects.

### Example 3

Domovictus 100 (a whey powder), Consense (a permeate powder obtained by ultrafiltration of whey), and Hiprotal 660 (a whey protein powder with approx. 60 % protein obtained as retentate with ultrafiltration) (all obtainable from Borculo Domo Ingredients) were tested using the above described protocol. The whey protein containing products all showed an anti-erosive effect as compared to the acidic beverages not containing the whey products; the best results were obtained with Consense, then with Domovictus 100, while the Hiprotal 660 fraction hardly had an effect.

### Example 4

Skimmed milk was subjected to ultrafiltration. The permeate was concentrated using freeze drying. The freeze-dried product was compared with the same amount of the whey permeate Consense. Both the milk permeate and the whey permeate were found to have an anti-erosive effect in the same order of magnitude.

### Example 5

A specific fraction was isolated by increasing the pH of a whey permeate solution of 20 % dry matter to 8.2 and heating said solution to 50°C for 1 hour. The precipitate was removed by microfiltration over a 0.8 micron filter. Both filtrate and precipitate were tested using the above described test protocol. The anti-erosive effect of the whey protein permeate was concentrated in the precipitate. This precipitate was treated at a pH of 3.0 to a size exclusion chromatography (gel filtration, Sephadex G-25) to remove salts. The salt free fraction has an anti-erosive effect.

### Example 6

To concentrate the active anti-erosive ingredients from permeate, a whey fraction with a high binding affinity for hydroxyapatite (a major component of tooth enamel) was isolated.

Particularly, 6.12 g Consense, 4 ml hydroxyapatite suspension, 12.5 ml 0.1M ammonia acetate buffer pH=4-5 were mixed and incubated at 25°C in a shaking stove for 20 hours. Then, the mixture was centrifuged and washed with water (five times). Phosphate buffer (25 ml, 0.5M, pH = 7.0) was subsequently added to the precipitate and during the next 4 hours desorption took place at room temperature. The supernatant was collected and dialysed with water during 24 hours. After this dialysis, the fraction was freeze dried. The freeze-dried product was tested for anti-erosive activity and was found to have distinctive anti-erosive effects.

### Example 7

In the standard etching fluid described above, calcium and/or phosphate ions were added in concentrations comparable to concentrations in Consense. It was found that the anti-erosive effect of the permeate was independent on the calcium and phosphate concentrations.

### Example 8

Deminal (demineralised whey powder) and Hiprotal (demineralised and delactosed whey powder) (both products available from Borculo Domo Ingredients) were tested on anti-erosive activity. The results indicate that both products, despite the demineralisation and delactosing step, still exibited anti-erosive effects. Deminal and Hiprotal are products not obtained by ultrafiltration; these products, however, do contain the permeate fraction, having very low calcium and phosphate values.

### Comparative Example 9

A commercially available casein phosphopeptide product (ex DMV, the Netherlands) was tested on anti-erosive fraction. No anti-erosive effect was found.

## Claims

1. Method for isolating a tooth-erosion reducing composition, comprising contacting of milk with hydroxyapatite; and eluting any materials attached to the hydroxyapatite.

2. The method of claim 1, comprising isolating the whey fraction of milk, and contacting of this whey fraction with hydroxyapatite.

3. The method of claim 1 or 2, wherein the milk or whey fraction is demineralised before contacting it with hydroxyapatite.

4. The method of any one of claims 1-3, wherein the milk or whey fraction is subjected to a lactose removing step before contacting it with hydroxyapatite.

5. Tooth erosion reducing composition obtainable by the method of any one of claims 1-4.

6. The tooth reducing composition of claim 5, obtained by subjecting milk to a step removing the proteins, and subjecting said protein free fraction to a concentrating step.

7. The tooth reducing composition of claim 5 or 6, obtained by subjecting the whey fraction of milk to a step removing the proteins; and subjecting said protein free fraction to a concentrating step.

8. The tooth reducing composition of claim 6 to 7, wherein the protein free fraction is subjected to a calcium-phosphate removing step, before the concentrating step.

9. Food composition, preferably a beverage, and more preferably an acidic beverage comprising the tooth reducing composition of any of claims 5-8.

10. Use of a whey fraction, not being milk minerals or lactose, for the manufacture of a composition to reduce tooth erosion.

11. The use of claim 10, wherein the composition is based on an acidic food product.

12. The use of claim 10 or 11, wherein the composition is a beverage.
